(19) Europäisches Patentamt
European Patent Office
Office européen des brevets

(11) **EP 4 535 000 A1**

(12) **EUROPEAN PATENT APPLICATION**
published in accordance with Art. 153(4) EPC

(43) Date of publication:
**09.04.2025 Bulletin 2025/15**

(21) Application number: **23825177.1**

(22) Date of filing: **17.08.2023**

(51) International Patent Classification (IPC):
***G01N 33/543*** (2006.01)    ***G01N 33/76*** (2006.01)

(52) Cooperative Patent Classification (CPC):
**G01N 33/76; G01N 33/54388**

(86) International application number:
**PCT/KR2023/012216**

(87) International publication number:
**WO 2025/037663 (20.02.2025 Gazette 2025/08)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Adtech Co., Ltd.**
**Gunpo-si, Gyeonggi-do 15845 (KR)**

(72) Inventors:
• **CHANG, Jin Dong**
**Seoul 03165 (KR)**
• **CHO, Sung Jin**
**Chungcheongnam-do 31112 (KR)**
• **KWAG, Won Jae**
**Gyeonggi-do 18374 (KR)**
• **KIM, Sang Jin**
**Seoul 07691 (KR)**

(74) Representative: **Kurig, Thomas**
**Becker Kurig & Partner**
**Patentanwälte mbB**
**Bavariastraße 7**
**80336 München (DE)**

(54) **NOVEL IMMUNODIAGNOSTIC DEVICE FOR DETERMINING NORMAL AND ABNORMAL PREGNANCIES BY MEASURING DISTRIBUTION RATIO OF BETA CORE FRAGMENT HCG**

(57) The present disclosure relates to an immunodiagnostic device for determining normal and abnormal pregnancy, and more particularly, to an immunodiagnostic device including a novel multi-binding anti-hCG monoclonal antibody that may recognize Intact hCG and βcf hCG in common, wherein Intact hCG and βcf hCG contained in the sample may be separately detected, including two detection lines and control lines, and the ratio of the color development intensity of βcf hCG to the color development intensity of Intact hCG and βcf hCG (the distribution ratio of βcf hCG) may be numerically quantified to determine the number of weeks of pregnancy, normal pregnancy, and abnormal pregnancy including ectopic pregnancy and blighted ovum accurately and quickly.

FIG. 1

**EP 4 535 000 A1**

**Description**

[TECHNICAL FIELD]

The present disclosure relates to an immunodiagnostic device for determining normal and abnormal pregnancy and an interpretation method using the same, and more particularly, to an immunodiagnostic device for determining normal and abnormal pregnancy and an interpretation method using the same by measuring a distribution ratio of βcf hCG (Beta core fragment hCG) among various hCGs secreted in pregnancy urine. The key principle of the immunodiagnostic device is to make a novel multi-binding anti-hCG monoclonal antibody that may bind by recognizing only Intact hCG and βcf hCG among hCG proteins present in various pregnancy urine as a mobile phase bound to a marker, and to specifically separate Intact hCG and βcf hCG in a fixed phase.

[BACKGROUND ART]

Human chorionic gonadotropin (hCG) is a glycoprotein hormone produced during pregnancy. hCG is produced in a chorionic trophoblast of placenta and continues to produce progesterone in an early stage of pregnancy, thus maintaining implantation until 10 weeks of pregnancy when the placental function is completed.

Complete hCG (Intact hCG; I-hCG) in an active form in a body has a molecular weight of about 37 kDa and is composed of 244 amino acids. Intact hCG is composed of two subunits, that is, alpha (α) subunit and beta (β) subunit. The alpha subunit contains 92 amino acids, and the beta subunit contains 145 amino acids. Intact hCG is generally present in blood during pregnancy and is the main structure that exhibits biological activity. In addition to the active form, various dissociation, and degradation products of hCG are found in blood and urine. These products include nicked hCG (N-hCG), free beta hCG (free β-hCG), free alpha hCG (free α-hCG), beta-core fragment hCG (β-core fragment hCG), and the like.

The beta core fragment hCG (βcf hCG) is an hCG protein that is mainly present in pregnancy urine and has a molecular weight of about 14 kDa. Intact hCG is predominantly secreted at 4 weeks of the last menstrual period (LMP), and βcf hCG starts to gradually increase from 5 weeks of the LMP and is secreted at a concentration that is superior to Intact hCG after 5 weeks and 6 days or 6 weeks (John Walter Larsen et al., 2015).

In the distribution of hCG protein in pregnant women, Intact hCG is dominant at 98% in blood, but βcf hCG increases up to 80% as the gestational weeks elapse and is present at a dominant concentration compared to Intact hCG. The conventional pregnancy diagnosis kit using Intact hCG as a marker determines the pregnancy by qualitatively diagnosing Intact hCG present in urine. However, as a result of testing 11,760 people who is judged to be pregnant with the conventional pregnancy diagnosis kit, 22 people (about 0.2%) are found to be false negative, and the Intact hCG blood concentration on them is measured at a maximum of 268,022.5 IU/mL, which indicates that the conventional urine pregnancy diagnosis kit has a false negative problem caused by the Hook effect. The fundamental reason for this is found to be caused by an excess of βcf hCG present in theurine (Richard T. Griffy et al., 2013).

In a urine pregnancy diagnosis, a false negative may have serious consequences in emergency department (ED) situations. For example, if a false negative result is obtained when measuring by a general pregnancy diagnosis kit using urine even though a female patient is pregnant, a medical accident may occur, in which a treatment is performed without considering the fetus in the treatment of the female patient.

Meanwhile, the ectopic pregnancy accounts for 9% of all deaths of pregnant women during the first trimester increases the mortality rate of pregnant women, and the prevalence of ectopic pregnancy is gradually increasing worldwide.

Under the circumstances, the present inventors conducted extensive research to solve the hook effect and develop a method for distinguishing between normal and abnormal pregnancies. As a result, the inventors develop a novel multi-binding anti-hCG monoclonal antibody that may recognize Intact hCG and βcf hCG in common, and solve the hook phenomenon by separating and detecting Intact hCG and βcf hCG in urine samples, respectively, and quantifying the ratio of the color development intensity of βcf hCG (the distribution ratio of βcf hCG) to the color development intensity of Intact hCG and βcf hCG, confirming that gestational weeks, normal pregnancy, and abnormal pregnancy including ectopic pregnancy and blighted ovum may be accurately and quickly identified.

[DETAILED DESCRIPTION OF THE INVENTION]

[TECHNICAL PROBLEM]

Embodiments of the inventive concept provide an immunodiagnostic device and an interpretation method for determining normal pregnancy and abnormal pregnancy in the early stage of pregnancy by specifically separating and detecting βcf hCG and Intact hCG contained in pregnancy urine, and accurately measuring βcf hCG distribution ratio.

[TECHNICAL SOLUTION]

The immunodiagnostic device for determining normal and abnormal pregnancy includes i) a sample area for accommodating a sample to be analyzed; ii) a conjugate area connected to the sample area, and including a multi-binding anti-hCG monoclonal antibody bonded to a probe material; iii) a signal detection area connected to the conjugate area and including a first detection line on which an anti-βcf hCG antibody that can specifically bind only to βcf hCG is fixed, a second detection line on which an anti-Intact hCG antibody that can specifically bind only to Intact hCG is fixed, and a control line; and iv) a moisture absorption area located on a downstream of the signal detection area that absorbs the sample to which the signal detection reaction is completed.

The method for analyzing information required to determine normal and abnormal pregnancy includes: i) binding by reflecting the distribution ratio of βcf hCG and Intact hCG in a sample to be analyzed, by using a multi-binding anti-hCG monoclonal antibody that recognizes βcf hCG and Intact hCG in common; ii) measuring a color development intensity of βcf hCG and Intact hCG; and iii) determining normal and abnormal pregnancy by an intensity distribution ratio of the color development of βcf hCG.

Advantages and features of the inventive concept and methods for achieving them will be apparent with reference to embodiments described below in detail in conjunction with the accompanying drawings. However, the inventive concept is not limited to the embodiments disclosed below, but can be implemented in various forms, and these embodiments are to make the disclosure of the inventive concept complete, and are provided so that this disclosure will be thorough and complete and will fully convey the scope of the invention to those of ordinary skill in the art, which is to be defined only by the scope of the claims.

The terminology used herein is for the purpose of describing particular embodiments only and is not intended to be limiting of the inventive concept. The singular expressions include plural expressions unless the context clearly dictates otherwise. In this specification, the terms "comprises" and/or "comprising" are intended to specify the presence of stated elements, but do not preclude the presence or addition of elements. Like reference numerals refer to like elements throughout the specification, and "and/or" includes each and all combinations of one or more of the mentioned elements. Although "first", "second", and the like are used to describe various components, these components are of course not limited by these terms. These terms are only used to distinguish one component from another. Thus, a first element discussed below could be termed a second element without departing from the teachings of the inventive concept.

Unless otherwise defined, all terms (including technical and scientific terms) used herein have the same meaning as commonly understood by one of ordinary skill in the art to which this invention belongs. It will be further understood that terms such as those defined in commonly used dictionaries, will not be interpreted in an idealized or overly formal sense unless expressly so defined herein.

The present disclosure provides an immunodiagnostic device and the interpretation method for determining normal pregnancy and abnormal pregnancy in the early stages of pregnancy by specifically separating and detecting βcf hCG and Intact hCG contained in pregnancy urine, and accurately measuring βcf hCG distribution ratio by using a multi-binding anti-hCG monoclonal antibody that recognizes Intact hCG and βcf hCG in common.

The multi-binding anti-hCG monoclonal antibody that recognizes Intact hCG and βcf hCG in common has the characteristic of accurately reflecting the distribution ratio of βcf hCG and Intact hCG according to the number of weeks of pregnancy and is used to determine normal pregnancy and abnormal pregnancy more accurately and efficiently according to the analysis of the distribution ratio.

An object of the present disclosure is to accurately determine normal and abnormal pregnancy. This is difficult to achieve by simply measuring Intact hCG and βcf hCG concentrations using the Intact hCG and βcf hCG antibodies, and may be achieved by measuring the ratio of the reaction between Intact hCG and βcf hCG present in a sample. In the case that the Intact hCG and βcf hCG antibodies are used, there is a limitation in the concentrations of Intact hCG and βcf hCG that may be recognized by the antibodies, so it is rather difficult to accurately determine the concentrations of Intact hCG and βcf hCG and the ratio. On the other hand, in the case that a monoclonal antibody that commonly recognizes Intact hCG and βcf hCG of the present disclosure is used, even in the case that there is a limitation to the values of Intact hCG and βcf hCG which are recognizable, since the ratio of reaction with Intact hCG and βcf hCG present in the sample is measured, the concentrations and the concentration ratio of Intact hCG and βcf hCG in the sample may be more accurately predicted.

According to the present disclosure, the monoclonal antibodies corresponding to Intact hCG and βcf hCG, respectively, are not used, but the monoclonal antibodies recognizing Intact hCG and βcf hCG in common are used. This is because the total Intact hCG and βcf hCG contents in a sample may be identified in proportion.

Using the multi-binding anti-hCG monoclonal antibody of the present disclosure, the accurate distribution ratio may be derived, and normal or abnormal pregnancy may be accurately determined.

In the present disclosure, the abnormal pregnancy may be ectopic pregnancy or blighted ovum, but not limited thereto.

The ectopic pregnancy refers to pregnancy that is conceived in a different place, not inside the uterus where a fertilized egg is normally conceived. The implantation position of the ectopic pregnancy may be an abnormal position in the uterus or may be somewhere other than the uterus. Most of them are oviduct pregnancy that occurs in the oviduct, and in rare cases, ovarian, peritoneal, and uterine tubular pregnancy. This is one of the most common emergency diseases and accounts for 1 to 2% of all pregnancies.

The blighted ovum refers to a case in which the placental tissue develops on ultrasound test, but the fetus is not visible. In the case of the blighted ovum, the placenta may grow for a while without an embryo, but it ends with a natural miscarriage in which the substances conceived in the uterus are discharged after bleeding and the lower abdominal pain.

Furthermore, the present disclosure provides an immunodiagnostic device for determining normal and abnormal pregnancy including i) a sample area for accommodating a sample to be analyzed; ii) a conjugate area connected to the sample area, and including a multi-binding anti-hCG monoclonal antibody bonded to a probe material; iii) a signal detection area connected to the conjugate area and including a first detection line on which an anti-βcf hCG antibody that can specifically bind only to βcf hCG is fixed, a second detection line on which an anti-Intact hCG antibody that can specifically bind only to Intact hCG is fixed, and a control line; and iv) a moisture absorption area located on a downstream of the signal detection area that absorbs the sample to which the signal detection reaction is completed.

It is preferable that i) the sample area accommodates a liquid sample such as a pregnant woman's urine, but any sample expected to contain Intact hCG and/or βcf hCG may be used.

The sample area may further have a filtering function to further improve selectivity to an analyzed material or to minimize an influence by an interference substance that may be included in the sample. If necessary, an auxiliary area including a material capable of increasing a reaction between the analyzed material and the conjugate or excluding an influence by an interference substance may be additionally provided at an upstream of the sample area.

The probe material in the conjugate area of ii) may be at least one selected from a group consisting of a gold nanoparticle, a silver nanoparticle, a quantum dot nanoparticle, a carbon nanoparticle, a latex bead/fluorescent nanoparticle, a cellulose nanoparticle, a magnetic nanoparticle, a silica nanoparticle, a polymer bead, a fluorescent material, a luminescent material, a pigment bead, and a protein, but not limited thereto.

In a specific embodiment of the present disclosure, the probe material may use a colloidal gold nanoparticle, but not limited thereto.

The signal detection area of iii) is a medium in which a mobile phase and a sample are deployed, and the mobile phase and the sample to be tested may move by a capillary phenomenon of the porous membrane of the signal detection area. The signal detection area may be any one selected from the group consisting of nitrocellulose, cellulose, polyethylene, polyethersulfone, polystyrene, polycarbonate, polymethyl methacrylate, nylon, PVDF, well plate synthesized with polyvinyl resin or polystyrene resin and slide glass made of glass, but is not limited thereto. In a specific embodiment of the present disclosure, the signal detection area may be a nitrocellulose membrane having pores of 5 to 15 μm, but not limited thereto.

According to a specific embodiment of the present disclosure, the signal detection area may include a first detection line immobilized with an anti-βcf hCG antibody, a second detection line immobilized with an anti-Intact hCG antibody downstream of the first detection line, and a control line downstream of the second detection line. According to another embodiment of the present disclosure, the signal detection area may include a second detection line immobilized with an anti-Intact hCG antibody, a first detection line immobilized with an anti-βcf hCG antibody downstream of the second detection line, and a control line downstream of the first detection line.

The anti-Intact hCG antibody is an antibody that specifically recognizes active Intact hCG in which two subunits of α and β are combined, and the anti-βcf hCG antibody is an antibody that specifically recognizes the beta core fragment hCG (βcf hCG).

The present disclosure relates to an immunodiagnostic device including two detection lines, and by quantitatively or qualitatively analyzing the color development intensity of the detection line that detects βcf hCG and Intact hCG in the sample, and quantifying and comparing the distribution ratio of βcf hCG through the following equation derived from the present disclosure, weeks of pregnancy, normal pregnancy, and abnormal pregnancy including ectopic pregnancy may be accurately determined.

$$\text{Beta core fragment hCG (\%)} = \frac{\beta\text{cf hCG Intensity}}{\text{Intact hCG Intensity} + \beta\text{cf hCG Intensity}} \times 100$$

In a specific embodiment of the present disclosure, in the case that the ratio of the color development intensity of βcf hCG to the color development intensity of βcf hCG and Intact hCG according to the above equation increases by 50 to 60% or more, it may be determined to be normal pregnancy, in the case that the ratio is 5 to 15% or less, it may be determined to be ectopic pregnancy, and in the case that the ratio is maintained for 6 weeks and 6 days at 20 to 30% or less, it may be determined to be blighted ovum.

The control line refers to a part of emitting a constant signal regardless of the concentration of Intact hCG or βcf hCG in the sample to be tested.

The moisture absorption area of iv) may include an absorbent dispersed in a pore of a porous support or adsorbed or coated on a fiber yarn of the porous support, and may further include a porous film layer on an upper surface of the porous support, but not limited thereto.

In the present disclosure, in the case that the multi-binding anti-hCG monoclonal antibody conjugate present in the conjugate area reacts with a sample such as urine of a pregnant woman, it reacts according to the concentration ratio of Intact hCG and βcf hCG in the sample, and the reacted conjugate moves toward the membrane. In the first detection line on the membrane to which the anti-βcf hCG monoclonal antibody is fixed, a sandwich-shaped complex is formed specifically for only βcf hCG among the hCGs that reacted to conjugate, and in the second detection line to which the anti-Intact hCG monoclonal antibody is fixed, a sandwich complex is formed specifically for Intact hCG among the hCGs that reacted to conjugate.

The immunodiagnostic device of the present disclosure may include a solid support thereunder. The solid support may be formed of any one material selected from the group consisting of nitrocellulose, nylon, PVDF, glass, and plastic, but is not limited thereto. Since a pad, a membrane, and the like may be attached to the solid support, durability of the strip may be improved, and handling and storage may be facilitated. In addition, mounting of additional external devices may be facilitated.

Plastic materials that may be used as the solid support may include a polypropylene film, a polyester film, a polycarbonate film, an acrylic film, and the like, but not limited thereto.

The immunodiagnostic device of the present disclosure may further include an analyzing device. The analyzing device may evaluate color development intensity of the first detection line and the second detection line. The analyzing device may include a reader combined with a software program capable of evaluating intensity, color, emission and/or fluorescence of the analyzed material complex formed on the first detection line and the second detection line. The reader may compare intensity, color, emission and/or fluorescence with a pre-programmed threshold value, and may provide a digital output based on the comparison. According to an embodiment of the present disclosure, the analyzing device uses a reflection reader equipped with analysis software based on a CCD camera or a laser light source, and normal pregnancy and abnormal pregnancy including ectopic pregnancy may be accurately determined with high accuracy. The analyzing device may further include a display that provides information related to abnormal pregnancy including ectopic pregnancy.

The immunodiagnostic device of the present disclosure may identify the number of weeks of pregnancy for observing a gestation sac and a fetus. In a specific embodiment of the present disclosure, in the case that the ratio of the color development intensity of βcf hCG to the color development intensity of βcf hCG and Intact hCG is increased by 50 to 60% or more at 5 to 6 weeks of the LMP, a gestation sac and a fetus may be observed as a normal pregnancy, whereas in the case that the ratio is 5 to 15% or less, a gestation sac may not be observed as ectopic pregnancy.

In another specific embodiment of the present disclosure, the ratio of the color development intensity of βcf hCG to the color development intensity of βcf hCG and Intact hCG, that is, βcf hCG / (βcf hCG + Intact hCG), is compared, and it was confirmed that abnormal pregnancy including ectopic pregnancy is significantly lower in comparison with normal pregnancy.

In addition, the present disclosure provides a kit in which the immunodiagnostic device is additionally fixed within a device.

The kit is provided with a guide and a strip support on a lower device, and a sample inlet and a result identification window at a location corresponding to the first detection line, the second detection line, and the control line on an upper device.

The upper and lower devices may be manufactured using a general plastic material, for example, a material such as polycarbonate, acrylonitrile butadiene styrene (ABS), and the like, but are not limited thereto.

In addition, the present disclosure provides a method for analyzing information required to determine normal and abnormal pregnancy including i) measuring an amount or concentration of βcf hCG and Intact hCG in a sample to be analyzed; and ii) determining normal and abnormal pregnancy by a ratio of the amount or concentration of βcf hCG with respect to the amount or concentration of βcf hCG and Intact hCG, wherein the amount or concentration is measured using a multi-binding anti-hCG monoclonal antibody that commonly recognizes βcf hCG and Intact hCG.

The amount or concentration may be measured by a color development intensity in proportional thereto.

The case in which a ratio of the color development intensity of βcf hCG with respect to the color development intensity of βcf hCG and Intact hCG exceeds 50% on 5 weeks and 6 days of the LMP may be determined to be normal pregnancy, and the case in which the ratio is 30% or less may be determined to be abnormal pregnancy including ectopic pregnancy and blighted ovum, but not limited thereto.

Specifically, the present disclosure provides a method for analyzing information required to determine normal and abnormal pregnancy including i) applying a sample to the immunodiagnostic device such that the Intact hCG and βcf hCG in the sample are reacted with a multi-binding anti-hCG monoclonal antibody bonded to a probe material; ii) identifying the reaction of βcf hCG and Intact hCG in the sample by a first detection line and a second detection line, in which βcf hCG and Intact hCG are detected by a color development intensity generated by a presence of a sandwich complex in each detection line; and iii) the case that the ratio of the color development intensity of the first detection line with respect to the first detection line and the second detection (the ratio of the color development intensity of βcf hCG to the color development intensities of βcf hCG and Intact hCG) increases by 50 to 60% or more on the LMP 5 to 6 weeks, that is, 5 weeks and 6 days of the LMP is determined to be normal pregnancy, the case that the ratio is 30% or less is determined to be ectopic pregnancy or blighted ovum.

In a specific embodiment of the present disclosure, it is identified that the concentration and the color development intensity are proportional by the color development intensity according to the concentrations of βcf hCG and Intact hCG.

Furthermore, the present disclosure provides a method for determining normal and abnormal pregnancy using an immunochromatography strip.

The method for determining normal and abnormal pregnancy may include i) applying a sample to the immunodiagnostic device such that the Intact hCG and βcf hCG in the sample are reacted with a multi-binding anti-hCG monoclonal antibody bonded to a probe material; ii) identifying the reaction of βcf hCG and Intact hCG in the sample by a first detection line and a second detection line, in which βcf hCG and Intact hCG are detected by a color development intensity generated by a presence of a sandwich complex in each detection line; and iii) the case that the ratio of the color development intensity of the first detection line with respect to the first detection line and the second detection (the ratio of the color development intensity of βcf hCG to the color development intensity of βcf hCG and Intact hCG) increases exceeding 50% on 5 weeks and 6 days of the LMP is determined to be normal pregnancy, and the case that the ratio is maintained for 5 weeks and 6 days at 30% or less is determined to be abnormal pregnancy.

[ ADVANTAGEOUS EFFECTS OF THE INVENTION]

The present disclosure provides a novel immunodiagnostic device for determining between normal and abnormal pregnancy by measuring the beta core fragment hCG (βcf hCG) distribution ratio, and an increase in the βcf hCG distribution ratio identified accordingly means that a fetus is normally conceived and grown, which will have the effect of relieving the anxiety of early pregnant women. In addition, if the βcf hCG distribution ratio is significantly lowered, prompt action and treatment in connection with a hospital may be expected, and this will help provide normal pregnancy maintenance information to a pregnant woman.

[ DESCRIPTION OF THE DRAWINGS]

FIG. 1 is a conceptual diagram illustrating the reaction principle caused by the concentration ratio of Intact hCG and βcf hCG in a sample of the present disclosure and showing an embodiment of an immunochromatography strip

FIG. 2 is a diagram illustrating the correlation of color development intensities for each amount or concentration of Intact hCG and βcf hCG.

FIG. 3 is a block diagram illustrating a distribution change of Intact hCG and βcf hCG in normal pregnancy.

FIG. 4 is a block diagram illustrating a distribution change of Intact hCG and βcf hCG in abnormal pregnancy.

FIG. 5 is a diagram of measuring and comparing a ratio of color development intensity of βcf hCG / (Intact hCG + βcf hCG) for normal and ectopic pregnancy groups by gestational week.

FIG. 6 is a block diagram illustrating a result of clinical evaluation of normal pregnancy sample using the diagnostic device of the present disclosure.

FIG. 7 is a block diagram illustrating a result of clinical evaluation of normal pregnancy sample using the diagnostic device of the present disclosure.

FIG. 8 is a block diagram illustrating a result of clinical evaluation of ectopic pregnancy sample using the diagnostic device of the present disclosure.

FIG. 9 is a block diagram illustrating a result of clinical evaluation of ectopic pregnancy sample using the diagnostic device of the present disclosure.

FIG. 10 is a diagram illustrating a result of the color development intensity correlation according to each concentration ratio when Intact hCG alone, βcf hCG alone, Intact hCG and βcf hCG mixed positive samples are prepared and reacted by concentration in a kit manufactured according to the present disclosure.

[ BEST MODE]

Hereinafter, the contents of the present disclosure will be described in more detail with the following embodiments and experimental examples. However, the scope of the present disclosure is not limited to the following embodiments and experimental examples, but includes modifications to the equivalent inventive concept.

## Embodiment 1. Preparation of immunochromatography strip

### 1-1. Fabrication of membrane formed with first detection line, second detection line, and control line.

Three different antibodies are dispensed onto a nitrocellulose membrane. Anti-βcf hCG monoclonal antibody as βcf hCG antibody is dispensed and dried on a first detection line. Anti-Intact hCG monoclonal antibody as Intact hCG antibody is dispensed and dried on a second detection line. Goat anti-mouse IgG is dispensed and dried onto a control line.

### 1-2. Fabrication of conjugate pad

A first conjugate solution containing colloidal gold nanoparticles and a multi-binding anti-hCG monoclonal antibody that recognizes Intact hCG and βcf hCG in common binding to the colloidal gold nanoparticles is prepared. A second conjugate solution containing colloidal gold nanoparticles and mouse immunoglobulin (Mouse IgG) binding to the colloidal gold nanoparticles is prepared. The first conjugate solution and the second conjugate solution are dispensed onto a pretreated conjugate pad and completely dried. Then, the pad is cut into an appropriate size.

### 1-3. Fabrication of sample pad

A sample pad is sufficiently soaked with a sample pad pretreatment solution containing a buffer and a preservative and completely dried, and then cut into an appropriate size.

### 1-4. Fabrication of absorption pad

A moisture absorption pad in a dry state is cut to an appropriate size.

### 1-5. Fabrication of immunochromatography strip

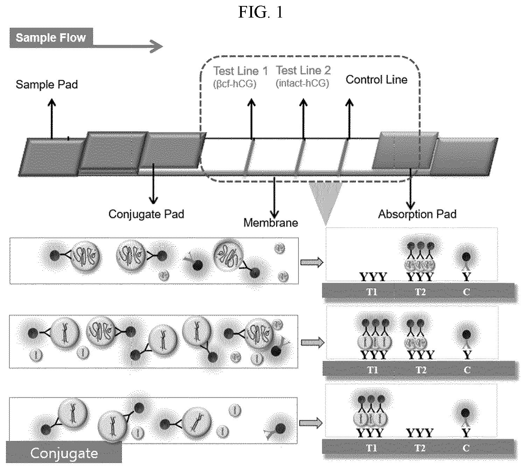

The membrane, the conjugate pad, the sample pad, and the moisture absorption pad, prepared in each of the processes are assembled in the structure as shown in FIG. 1.
That is, the sample pad is attached to overlap one end of the conjugate pad, one end of the sample pad is attached to overlap the other end of the conjugate pad, and the other end of sample pad is attached overlap one end of the moisture absorption pad.
In FIG. 1, the immunochromatography strip has the following structure.

1. Sample pad
2. Conjugate pad
3. Nitrocellulose membrane
4. Moisture absorption pad
5. First detection line (Test line 1) to which anti-βcf hCG monoclonal antibody is fixed.
6. Second detection line (Test line 2) to which anti-Intact hCG monoclonal antibody is fixed.
7. Control line to which goat anti-mouse IgG is fixed.

### 1-6. Analyzing device

A reflectance reader equipped with analysis software based on a CCD camera or laser light source is used to quantify the color development or contrast sensitivity of the detection line and to quantify it as signal strength.

### 1-7. Device Assembly

The prepared immunochromatography strip for determining normal pregnancy, gestational weeks, and abnormal pregnancy including ectopic pregnancy and blighted ovum is inserted into a strip fixing position of the plastic lower device, and then, inserted into the upper device having a sample inlet and a result checking window.

## Embodiment 2. Measurement of change in color development intensity according to concentration of Intact hCG and βcf hCG in normal pregnancy group and abnormal pregnancy group including ectopic pregnancy

Conventionally, a diagnosis has been made based on a concentration ratio, but according to the pregnancy diagnostic device of the present disclosure, a diagnosis is determined by identifying the color development intensity. The color development intensity is measured and quantified in the same manner in all devices measuring the color development intensity.

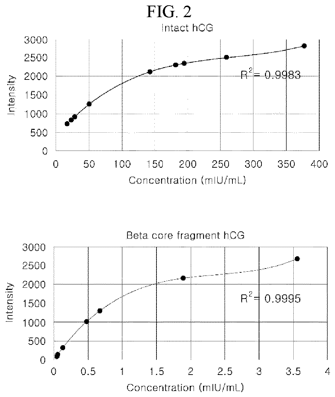

The measurement is performed using the analyzing device, which is a component of the pregnancy diagnostic device of the present disclosure, and it is identified that the concentration and the color development intensity are proportional (FIG. 2).

### 2-1. Measurement of change in color development intensity according to concentration of Intact hCG and βcf hCG in normal pregnancy group

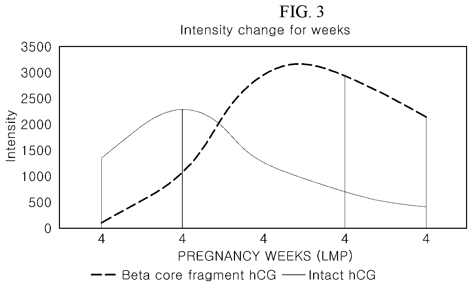

The change in the color development intensity according to the concentration of Intact hCG and βcf hCG is measured in a normal pregnancy group. In the normal pregnancy group, Intact hCG starts to be detected from 4 days after 3 weeks of the LMP and increases to 5 weeks. Accordingly, it is possible to test an early pregnancy. βcf hCG starts to be detected from the late 4 weeks of the LMP, increases rapidly from 5 weeks, and is maintained at a high concentration after 6 weeks (FIG. 3).

In addition, when βcf hCG is detected at 5 weeks of the LMP, a gestation sac (baby house) may be identified as a result of an ultrasonic test. From 6 weeks of the LMP, Intact hCG is decreased, and βcf hCG is maintained, and a reversal phenomenon occurs, and as a result of an ultrasonic test, a fetus may be identified.

### 2-2. Measurement of change in color development intensity according to concentration of Intact hCG and βcf hCG in abnormal pregnancy group including ectopic pregnancy

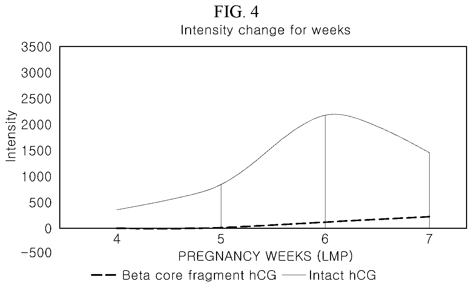

As a result of measuring the amount of change in Intact hCG and βcf hCG concentration in the ectopic pregnancy group, Intact hCG is detected at 5 weeks of the LMP, but βcf hCG is not detected at 5 weeks of the LMP or at a very low concentration (FIG. 4).

As a result, in the normal pregnancy between 4 and 7 weeks of the LMP, the concentration of Intact hCG increases and then decreases, and the concentration of βcf hCG gradually increases and maintains. However, in the case of ectopic pregnancy, βcf hCG is undetected or detected at a very low concentration, confirming that this is a significant result that distinguishes between normal pregnancy and ectopic pregnancy.

On the other hand, in the case of blighted ovum, βcf hCG remains low without increasing by less than 30% from 5 weeks and 6 days of the LMP, and an ultrasonic observation confirms that a gestation sac is identified, but a fetus and heart sound are not identified.

### Embodiment 3. Measurement of color development intensity ratio of βcf hCG / (Intact hCG + βcf hCG) in normal pregnancy group and abnormal pregnancy group including ectopic pregnancy

To use the result of embodiment 2 described above for a determination of gestational weeks, a normal pregnancy group and an abnormal pregnancy group including ectopic pregnancy, the color development intensity ratio according to the concentration for each gestational week of βcf hCG / (Intact hCG + βcf hCG) is measured and compared for the normal pregnancy group and the abnormal pregnancy group.

### 3-1. Verification of measurement principle and derivation of equation of βcf hCG distribution ratio (%)

The pregnancy diagnostic device of the present disclosure has a "mobile phase" that reacts with the sample to move to a fixed phase and the "fixed phase" capable of separately detecting Intact hCG and βcf hCG present in the sample. In the present disclosure, the mobile phase corresponds to the conjugate area among the components of the pregnancy diagnostic device, and the fixed phase corresponds to the signal detection area including the first detection line, the second detection line, and the control line.

The mobile phase is in a dried state of combining an indicator that may express the concentration intensity of the sample, that is, a gold particle, a nano bead, fluorescence, or phosphorescence, with a multi-binding anti-hCG monoclonal antibody that may recognize Intact hCG and βcf hCG in common, which is novel antibody developed by the present inventors.

In other words, the characteristics of the antibody bound to the marker are reacting by recognizing Intact hCG and βcf hCG in common.

Anti-βcf hCG monoclonal antibody is fixed in the first detection line of the fixed phase, and only βcf hCG is detected in the sample, and anti-Intact hCG monoclonal antibody is fixed in the second detection line, and accordingly, only Intact hCG is detected in the sample.

Accordingly, the sample reacts primarily with the conjugate according to the concentration ratio of Intact hCG and βcf hCG contained in the sample and is shifted to the fixed phase, and each sample is separated and detected according to the characteristics of the antibody fixed on the fixed phase.

On the other hand, the equation of the βcf hCG distribution ratio (%) is derived from the color development intensity ratio of βcf hCG / (Intact hCG + βcf hCG), which made it possible to measure and analyze the sample more clearly.

**3-2. Measurement of color development intensity ratio of βcf hCG / (Intact hCG + βcf hCG) in normal pregnancy group and abnormal pregnancy group including ectopic pregnancy for each of gestational weeks**

The distribution ratio (%) of βcf hCG from the color development intensity ratio of βcf hCG / (Intact hCG + βcf hCG) is identified through the following equation based on the color development intensity according to the detected concentration ratio of Intact hCG and βcf hCG measured in a normal pregnancy group and an abnormal pregnancy group including ectopic pregnancy for each of gestational weeks.

$$\text{Beta core fragment hCG (\%)} = \frac{\beta cf\ hCG\ Intensity}{Intact\ hCG\ Intensity + \beta cf\ hCG\ Intensity} \times 100$$

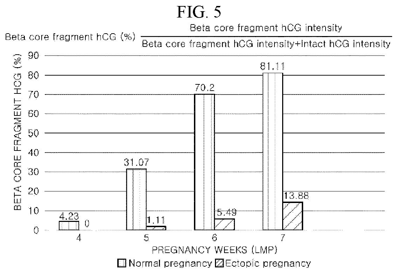

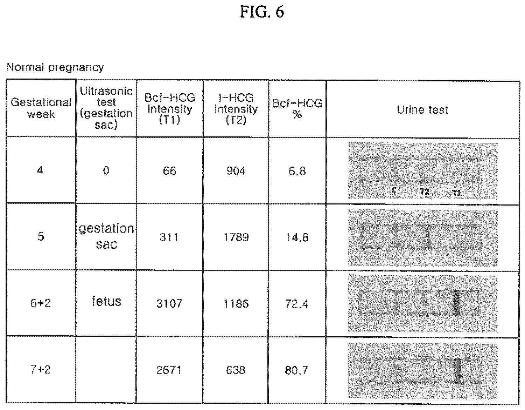

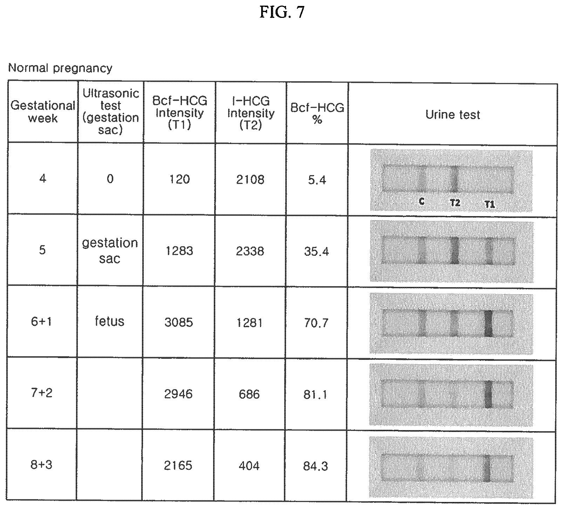

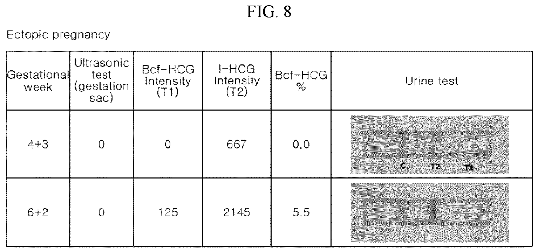

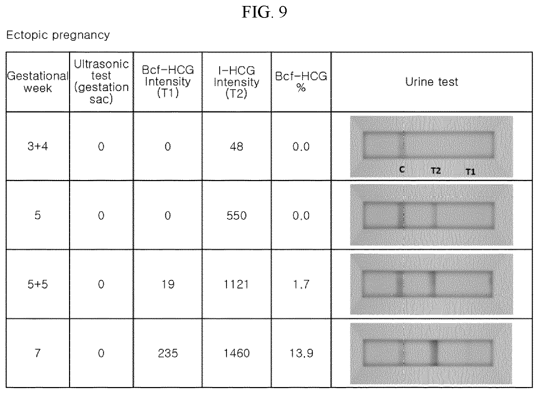

As a result, as shown in Table 1 and FIGS. 5 to 9, in the case of normal pregnancy in which a fertilized egg is normally implanted in the uterus and a baby grows, the distribution ratio of βcf hCG is about 10 to 20% at 5 weeks 0 of the LMP, increases to 50% at 5 weeks 6 days of the LMP, and continues at a rate of 70% at 6 weeks of LMP and 80% after 8 weeks of the LMP. On the other hand, in the case of abnormally conceived ectopic pregnancy, it is confirmed that the distribution ratio of βcf hCG at 5 weeks to 6 weeks of the LMP is maintained at 10% or less, the increase phenomenon may not be observed, and the gestation sac (baby center) is not observed on an ultrasonic test.

[Table 1]

| Intensity ratio comparison for gestational weeks between normal pregnancy and ectopic pregnancy Unit (%) | | | | | | |
|---|---|---|---|---|---|---|
| | 3rd week | 4th week | 5th week | 6th week | 7th week | 8th week |
| Ectopic pregnancy | 0 | 0 | 1.11 | 5.49 | 13.88 | - |
| Normal pregnancy | 4.98 | 4.23 | 31.07 | 70.02 | 81.11 | 84.27 |
| ** Percentage % according to each week is shown based on a median value. | | | | | | |

In addition, in the case of blighted ovum, it is confirmed that the distribution ratio of βcf hCG does not increase from 5 weeks and 6 days of the LMP and is maintained below 30%. As a result of an ultrasonic observation, the gestation sac is identified, but a fetus and heart sound are not identified.

Accordingly, the pregnancy diagnostic device of the present disclosure may distinguish between the normal pregnancy group and the abnormal pregnancy group including ectopic pregnancy based on the color development intensity ratio of βcf hCG / (Intact hCG + βcf hCG), and the distribution ratio (%) of βcf hCG based on the color development intensity according to the detected concentration of Intact hCG and βcf hCG, and may identify the timing when gestation sac is observed more accurately and quickly with one measurement.

**Embodiment 4. Kit performance (specificity) verification and color development intensity correlation analysis**

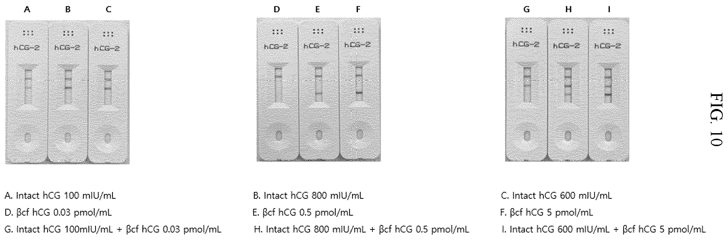

Based on the kit manufactured according to the present disclosure, the results of manufacturing and reacting Intact hCG alone, βcf hCG alone, Intact hCG and βcf hCG mixed positive samples by concentration are shown in FIG. 10.

As a result of the manufacturing positive sample by diluting Intact hCG to 100, 600, and 800 mIU/mL concentrations in a negative standard sample and reaction thereof, the first detection line (βcf hCG) does not develop color, and the color development intensity by concentration is identified on the second detection line (Intact hCG).

As a result of the manufacturing positive sample by diluting βcf hCG to 0.03, 0.5, and 5 pmol/mL concentrations in a negative standard sample and reaction thereof, the second detection line (Intact hCG) does not develop color, and the color development intensity for each concentration is identified in the first detection line (βcf hCG).

As a result of mixing and diluting the Intact hCG and βcf hCG in a negative standard sample, 100 mIU/mL (Intact hCG) + 0.03 pmol/mL (βcf hCG), 800 mIU/mL (Intact hCG) + 0.5 pmol/mL (βcf hCG), 600 mIU/mL (Intact hCG) + 5 pmol/mL (βcf hCG) to prepare a positive sample and react, both the first and second detection lines show the color development intensity corresponding to each concentration, and the color development intensity is equal for each concentration as a result of treatment with Intact hCG alone and βcf hCG alone.

These results verify that the multi-binding anti-hCG monoclonal antibody used in the present disclosure responds specifically to Intact hCG and βcf hCG without interference, suggesting that the color development intensity increases in a quantitative correlation according to the concentration of Intact hCG and βcf hCG mixed in the sample, allowing accurate distribution ratio to be measured.

While the inventive concept has been described with reference to embodiments, it will be apparent to those skilled in the art that various changes and modifications may be made without departing from the spirit and scope of the inventive concept. Therefore, it should be understood that the above embodiments are not limiting, but illustrative.

**Claims**

1. An immunodiagnostic device for determining normal and abnormal pregnancy, comprising:

    i) a sample area for accommodating a sample to be analyzed;
    ii) a conjugate area connected to the sample area, and including a multi-binding anti-hCG monoclonal antibody that recognizes Intact hCG and βcf hCG, and bonded to a probe material in common;
    iii) a signal detection area, in which Intact hCG and βcf hCG bonded in response to the conjugate are completely separated and detected, and including a first detection line on which a monoclonal antibody that forms a complex specifically to βcf hCG is fixed, a second detection line on which a monoclonal antibody that forms a complex specifically to Intact hCG is fixed, and a control line; and
    iv) a moisture absorption area located on a downstream of the signal detection area that absorbs the sample to which the signal detection reaction is completed.

2. The immunodiagnostic device according to claim 1, further comprising an analyzing device.

3. The immunodiagnostic device according to claim 1 or 2, wherein the normal and abnormal pregnancy are determined by a ratio of a color development intensity of βcf hCG with respect to a color development intensity of βcf hCG and Intact hCG measured in the detection lines derived by the following equation:

$$\text{Beta core fragment hCG (\%)} = \frac{\beta\text{cf hCG Intensity}}{\text{Intact hCG Intensity} + \beta\text{cf hCG Intensity}} \times 100.$$

4. The immunodiagnostic device according to claim 1, wherein the probe material of ii) is at least one selected from a group consisting of a gold nanoparticle, a silver nanoparticle, a quantum dot nanoparticle, a carbon nanoparticle, a latex bead/fluorescent nanoparticle, a cellulose nanoparticle, a magnetic nanoparticle, a silica nanoparticle, a polymer bead, a fluorescent material, a luminescent material, a pigment, and a protein.

5. The immunodiagnostic device according to claim 1, wherein the signal detection area of iii) is any one selected from a group consisting of nitrocellulose, cellulose, polyethylene, polyethersulfone, and nylon.

6. The immunodiagnostic device according to claim 1, wherein the moisture absorption area of iv) includes an absorbent dispersed in a pore of a porous support or adsorbed or coated on a fiber yarn of the porous support.

7. The immunodiagnostic device according to claim 1, wherein the immunodiagnostic device is capable of checking a number of weeks of pregnancy in which a gestation sac is observable by a color development of the first detection line.

8. A method for analyzing information required to determine normal and abnormal pregnancy, comprising:

    i) measuring an amount or concentration of βcf hCG and Intact hCG in a sample to be analyzed; and
    ii) determining normal and abnormal pregnancy by a ratio of the amount or concentration of βcf hCG with respect to the amount or concentration of βcf hCG and Intact hCG, wherein the amount or concentration is measured using a multi-binding anti-hCG monoclonal antibody that commonly recognizes βcf hCG and Intact hCG.

9. The method according to claim 8, wherein the amount or concentration is measured by a color development intensity in proportional thereto.

10. The method according to claim 8 or 9, wherein a case in which a ratio of the color development intensity of βcf hCG with respect to the color development intensity of βcf hCG and Intact hCG exceeds 50% on 5 weeks and 6 days of LMP is determined to be the normal pregnancy, and a case in which the ratio is 30% or less is determined to be the abnormal pregnancy including ectopic pregnancy and blighted ovum.

FIG. 1

FIG. 2

Intact hCG

$R^2 = 0.9983$

Beta core fragment hCG

$R^2 = 0.9995$

FIG. 3

Intensity change for weeks

FIG. 4

Intensity change for weeks

- - Beta core fragment hCG ——— Intact hCG

FIG. 5

Beta core fragment hCG (%) = Beta core fragment hCG intensity / (Beta core fragment hCG intensity + Intact hCG intensity)

□ Normal pregnancy ⊠ Ectopic pregnancy

FIG. 6

Normal pregnancy

| Gestational week | Ultrasonic test (gestation sac) | Bcf–HCG Intensity (T1) | I–HCG Intensity (T2) | Bcf–HCG % | Urine test |
|---|---|---|---|---|---|
| 4 | 0 | 66 | 904 | 6.8 | |
| 5 | gestation sac | 311 | 1789 | 14.8 | |
| 6+2 | fetus | 3107 | 1186 | 72.4 | |
| 7+2 | | 2671 | 638 | 80.7 | |

FIG. 7

Normal pregnancy

| Gestational week | Ultrasonic test (gestation sac) | Bcf–HCG Intensity (T1) | I–HCG Intensity (T2) | Bcf–HCG % | Urine test |
|---|---|---|---|---|---|
| 4 | 0 | 120 | 2108 | 5.4 | |
| 5 | gestation sac | 1283 | 2338 | 35.4 | |
| 6+1 | fetus | 3085 | 1281 | 70.7 | |
| 7+2 | | 2946 | 686 | 81.1 | |
| 8+3 | | 2165 | 404 | 84.3 | |

FIG. 8

Ectopic pregnancy

| Gestational week | Ultrasonic test (gestation sac) | Bcf–HCG Intensity (T1) | I–HCG Intensity (T2) | Bcf–HCG % | Urine test |
|---|---|---|---|---|---|
| 4+3 | 0 | 0 | 667 | 0.0 | |
| 6+2 | 0 | 125 | 2145 | 5.5 | |

FIG. 9

Ectopic pregnancy

| Gestational week | Ultrasonic test (gestation sac) | Bcf-HCG Intensity (T1) | I-HCG Intensity (T2) | Bcf-HCG % | Urine test |
|---|---|---|---|---|---|
| 3+4 | 0 | 0 | 48 | 0.0 | |
| 5 | 0 | 0 | 550 | 0.0 | |
| 5+5 | 0 | 19 | 1121 | 1.7 | |
| 7 | 0 | 235 | 1460 | 13.9 | |

FIG. 10

A. Intact hCG 100 mIU/mL
B. Intact hCG 800 mIU/mL
C. Intact hCG 600 mIU/mL
D. βcf hCG 0.03 pmol/mL
E. βcf hCG 0.5 pmol/mL
F. βcf hCG 5 pmol/mL
G. Intact hCG 100mIU/mL + βcf hCG 0.03 pmol/mL
H. Intact hCG 800 mIU/mL + βcf hCG 0.5 pmol/mL
I. Intact hCG 600 mIU/mL + βcf hCG 5 pmol/mL

## INTERNATIONAL SEARCH REPORT

| International application No. |
|---|
| **PCT/KR2023/012216** |

### A. CLASSIFICATION OF SUBJECT MATTER
**G01N 33/543**(2006.01)i; **G01N 33/76**(2006.01)i

According to International Patent Classification (IPC) or to both national classification and IPC

### B. FIELDS SEARCHED

Minimum documentation searched (classification system followed by classification symbols)

G01N 33/543(2006.01); A61B 10/00(2006.01); A61B 5/00(2006.01); G01N 33/53(2006.01); G01N 33/558(2006.01); G01N 33/574(2006.01); G01N 33/76(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 임신(pregnancy), 진단 장치(test device), 베타 코어 단편 hCG(beta core fragment hCG)

### C. DOCUMENTS CONSIDERED TO BE RELEVANT

| Category* | Citation of document, with indication, where appropriate, of the relevant passages | Relevant to claim No. |
|---|---|---|
| X | KR 10-2006-0121315 A (HUMASIS CO., LTD.) 29 November 2006 (2006-11-29)<br>See abstract; claims 1-2 and 13-14; pages 7-10 and 15; tables 1-3; and example 5. | 1-10 |
| A | KR 10-2079738 B1 (ADTECH CO., LTD.) 20 February 2020 (2020-02-20)<br>See claims 1-6. | 1-10 |
| A | KR 10-2473036 B1 (ADTECH CO., LTD.) 01 December 2022 (2022-12-01)<br>See entire document. | 1-10 |
| A | KR 10-2138192 B1 (PROTEOMETECH INC.) 27 July 2020 (2020-07-27)<br>See entire document. | 1-10 |
| A | KR 10-2002-0014004 A (HUMASIS CO., LTD.) 25 February 2002 (2002-02-25)<br>See entire document. | 1-10 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **14 May 2024** | **14 May 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT
Information on patent family members

International application No.

**PCT/KR2023/012216**

| Patent document cited in search report | | | Publication date (day/month/year) | Patent family member(s) | | | Publication date (day/month/year) |
|---|---|---|---|---|---|---|---|
| KR | 10-2006-0121315 | A | 29 November 2006 | EP | 1883816 | A1 | 06 February 2008 |
| | | | | US | 2009-0208983 | A1 | 20 August 2009 |
| | | | | US | 8101369 | B2 | 24 January 2012 |
| | | | | WO | 2006-126821 | A1 | 30 November 2006 |
| KR | 10-2079738 | B1 | 20 February 2020 | JP | 2021-532332 | A | 25 November 2021 |
| | | | | JP | 7144448 | B2 | 29 September 2022 |
| | | | | US | 2021-0003568 | A1 | 07 January 2021 |
| | | | | WO | 2021-002528 | A1 | 07 January 2021 |
| KR | 10-2473036 | B1 | 01 December 2022 | KR | 10-2021-0103976 | A | 24 August 2021 |
| KR | 10-2138192 | B1 | 27 July 2020 | CN | 113227791 | A | 06 August 2021 |
| | | | | EP | 3904878 | A1 | 03 November 2021 |
| | | | | EP | 3904878 | B1 | 21 February 2024 |
| | | | | KR | 10-2020-0082208 | A | 08 July 2020 |
| | | | | US | 2022-0146509 | A1 | 12 May 2022 |
| | | | | WO | 2020-138614 | A1 | 02 July 2020 |
| KR | 10-2002-0014004 | A | 25 February 2002 | AU | 2001-78808 | A1 | 25 February 2002 |
| | | | | AU | 7880801 | A | 25 February 2002 |
| | | | | CN | 1446064 | A | 01 October 2003 |
| | | | | EP | 1307134 | A1 | 07 May 2003 |
| | | | | EP | 1307134 | B1 | 10 April 2013 |
| | | | | JP | 2004-506220 | A | 26 February 2004 |
| | | | | KR | 10-0403871 | B1 | 01 November 2003 |
| | | | | US | 2003-0153093 | A1 | 14 August 2003 |
| | | | | US | 7332350 | B2 | 19 February 2008 |
| | | | | WO | 02-13685 | A1 | 21 February 2002 |